# EUROPEAN PATENT SPECIFICATION

(11) **EP 4 144 337 B1**
(45) Date of publication and mention of the grant of the patent: **07.08.2024**
(21) Application number: 21020442.6
(22) Date of filing: 02.09.2021
(51) Int. Cl.: A61K 8/11, A61K 8/64, A61K 8/9783

(54) **VITIS VINIFERA COSMETIC COMPOSITION**
KOSMETISCHE ZUSAMMENSETZUNG AUS VITIS VINIFERA
COMPOSITION COSMÉTIQUE DE VITIS VINIFERA

(43) Date of publication of application: 08.03.2023
(73) Proprietor: Guangzhou Jiyan Cosmetics Technology Co. Ltd., Guangzhou, Guangzhou Province (CN)
(72) Inventor: HE, Felix, Guangzhou, Guangzhou Province (CN); CAI, Zhuo, Guangzhou, Guangzhou Province (CN); ENNAMANY, Rachid, 3300 Bordeaux (FR)
(74) Representative: De Clercq & Partners

(56) References cited:
- EP-B1- 1 485 064
- WO-A1-2015/162052
- WO-A1-2017/100421
- PL-A1- 434 339
- US-A1- 2009 208 544

### SEQUENCE LISTING

<110> Guangzhou Jiyan Cosmetics Technology Co. LTD.
<120> Vitis vinifera cosmetic composition
<130> RP/Guang/21-02
<160> 24
<170> BiSSAP 1.3.6
<210> 1
<211> 6
<212> PRT
<213> Homo sapiens
<220>
<223> acetyl hexapeptide-8
<400> 1
   Glu Glu Met Gln Arg Arg
   1 5
<210> 2
<211> 6
<212> PRT
<213> artificial sequence
<220>
<223> synthetic peptide
<400> 2
   Gln Glu Met Arg Met Gln
   1 5
<210> 3
<211> 6
<212> PRT
<213> artificial sequence
<220>
<223> synthetic peptide
<400> 3
   Asp Glu Met Arg Met Gln
   1 5
<210> 4
<211> 6
<212> PRT
<213> artificial sequence
<220>
<223> synthetic peptide
<400> 4
   Glu Glu Met Arg Met Gln
   1 5
<210> 5
<211> 6
<212> PRT
<213> artificial sequence
<220>
<223> synthetic peptide
<400> 5
   Gln Gln Met Arg Met Gln
   1 5
<210> 6
<211> 6
<212> PRT
<213> artificial sequence
<220>
<223> synthetic peptide
<400> 6
   Gln Glu Met Lys Met Gln
   1 5
<210> 7
<211> 6
<212> PRT
<213> artificial sequence
<220>
<223> synthetic peptide
<400> 7
   Gln Glu Met Arg Met Glu
   1 5
<210> 8
<211> 6
<212> PRT
<213> artificial sequence
<220>
<223> synthetic peptide
<400> 8
   Gln Glu Met Arg Met Asn
   1 5
<210> 9
<211> 6
<212> PRT
<213> artificial sequence
<220>
<223> synthetic peptide
<400> 9
   Glu Glu Met Arg Met Glu
   1 5
<210>10
<211> 6
<212> PRT
<213> artificial sequence
<220>
<223> synthetic peptide
<400> 10
   Gln Asp Met Arg Met Gln
   1 5
<210> 11
<211> 6
<212> PRT
<213> artificial sequence
<220>
<223> synthetic peptide
<400> 11
   Gln Glu Leu Arg Met Gln
   1 5
<210>12
<211> 6
<212> PRT
<213> artificial sequence
<220>
<223> synthetic peptide
<400> 12
   Gln Glu Ile Arg Met Gln
   1 5
<210> 13
<211> 6
<212> PRT
<213> artificial sequence
<220>
<223> synthetic peptide
<400> 13
   Gln Glu Met Arg Ile Gln
   1 5
<210>14
<211> 6
<212> PRT
<213> artificial sequence
<220>
<223> synthetic peptide
<400> 14
   Gln Glu Met Arg Leu Gln
   1 5
<210> 15
<211> 6
<212> PRT
<213> artificial sequence
<220>
<223> synthetic peptide
<400> 15
   Glu Glu Ile Arg Met Gln
   1 5
<210> 16
<211> 6
<212> PRT
<213> artificial sequence
<220>
<223> synthetic peptide
<400>16
   Asp Gln Met Arg Met Gln
   1 5
<210> 17
<211> 6
<212> PRT
<213> artificial sequence
<220>
<223> synthetic peptide
<400> 17
   Glu Gln Met Lys Met Gln
   1 5
<210> 18
<211> 6
<212> PRT
<213> artificial sequence
<220>
<223> synthetic peptide
<400> 18
   Gln Asp Met Arg Met Asn
   1 5
<210> 19
<211> 6
<212> PRT
<213> artificial sequence
<220>
<223> synthetic peptide
<400> 19
   Asn Glu Ile Arg Val Gln
   1 5
<210> 20
<211> 6
<212> PRT
<213> artificial sequence
<220>
<223> synthetic peptide
<400> 20
   Asn Glu Ile Arg Val Gln
   1 5
<210> 21
<211> 6
<212> PRT
<213> artificial sequence
<220>
<223> synthetic peptide
<400> 21
   Gln Asp Met Lys Met Asn
   1 5
<210> 22
<211> 6
<212> PRT
<213> artificial sequence
<220>
<223> synthetic peptide
<400> 22
<210> 23
<211> 6
<212> PRT
<213> artificial sequence
<220>
<223> synthetic peptide
<400> 23
<210> 24
<211> 6
<212> PRT
<213> artificial sequence
<220>
<223> synthetic peptide
<400> 24

## Description

The present invention relates to a cosmetic composition suitable for inhibiting interleukin inflammation mediators IL-1α and IL-6 at the human skin level, while modulating the human Beta Defensin 2 at the human skin level.

Skin inflammation, for example due to UVB irradiation is a cause of skin aging and thus also a cause for premature wrinkles formation.

The cosmetic composition of the invention is thus also an anti-aging cosmetic composition and an anti-wrinkles cosmetic composition, such as for reducing and/or eliminating wrinkles or other aging effects from the skin. The composition of the invention is thus a composition suitable for protecting the skin from UVB induced inflammatory process, and for protecting the skin from bacterial attacks.

The cosmetic composition of the invention comprises dedifferentiated elicited Vitis vinifera cells and specific hexapeptide compound(s).

WO2015162052 teaches preparation of dediferentiated elicited cells of Bougamvillier. EP1892247 discloses a dermo-cosmetic composition comprising enkephalin-derived peptides for reducing and/or eliminating facial wrinkles. The composition comprises at least a peptide of the following general formula: 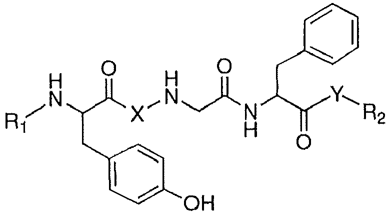 and at least an effective amount of at least one extract with anti-wrinkle and/or anti-ageing activity, such as Vitis vinifera, Rosa canina, Curcuma longa, Iris pallida, ..., argireline (R), Myoxinol (R), Kollaren (R), etc.

No example is given in EP1892247 for a cosmetic composition comprising an extract of Vitis vinifera and argireline (R). No suggestion is given in WO 2017/100421 to test a cosmetic composition comprising an extract of Vitis vinifera and argireline (R), especially a cosmetic composition of the invention.

WO2017/100421 discloses different hexapeptide compounds useful in the treatment and/or care of the skin, hair, nails and/or mucous membrane, said hexapeptide compounds being useful as skin anti-aging agents and, in particular as skin rejuvenating agents. Said document discloses also cosmetic composition comprising said hexapeptide compound and a cosmetic active agent, like anti-wrinkle agent and/or anti-aging agent, which can be selected amoung a large group of compounds, among others extracts or hydrolyzed extracts of Vitis vinifera.

No reference is made in WO2017/100421 to the use of cells of Vitis vinifera enriched with hexapeptide compound in a cosmetic composition, whereby said composition is suitable for inhibiting interleukin inflammation mediators IL-1α and IL-6 at the human skin level, while modulating the human Beta Defensin 2 at the human skin level.

The cosmetic composition of the invention comprises a cosmetic effective amount of at least dedifferentiated elicited Vitis vinifera cells homogeneously distributed in a cosmetic acceptable support, in which the dedifferentiated elicited Vitis vinifera cells, separated the one form the other and/or in cell agglomerates with a particle size of less than 0.5mm separated the one from the other, are dispersed, whereby the dedifferentiated elicited Vitis vinifera cells are enriched with at least a hexapeptide compound of the general formula (I):

Rₗ-Wₘ -Xₙ-AA1-AA2 -AA3-AA4-AA5-AA6-Yₚ-Z_{q}-R₂ (I)

its stereoisomers and/or its cosmetically or pharmaceutically acceptable salts, characterized in that:
AA1 is selected from the group formed by Gln, Glu, Asp and Asn;
AA2 is selected from the group formed by Glu, Gln and Asp;
AA3 is selected from the group formed by Met, Ile and Leu;
AA4 is selected from the group consisting of (a) Arg, Lys and Orn for any AA1, AA2 and AA3, and (b) Gln in case AA1, AA2 and AA3 are respectively Glu, Glu and Met;
AA5 is selected from the group consisting of (a) Met, Leu, Ile and Val for any AA1, AA2 and AA3, and (b) Arg in case AA1, AA2 and AA3 are respectively Glu, Glu and Met;
AA6 is selected from the group consisting of (a) Gln, Asn and Glu for any AA1, AA2 and AA3, and (b) Arg in case AA1, AA2 and AA3 are respectively Glu, Glu and Met;
W, X, Y and Z are each independently an amino acid; m, n, p and q are each independently 0 or 1 ;
Rₗ is selected from the group formed by H, a polymer derived from polyethylene glycol, substituted or unsubstituted non-cyclic aliphatic group, substituted or unsubstituted alicyclyl, substituted or unsubstituted heterocyclyl, substituted or unsubstituted heteroarylalkyl, substituted or unsubstituted aryl, substituted or unsubstituted aralkyl and R₅-CO-, wherein R₅ is selected from the group formed by H, substituted or unsubstituted non-cyclic aliphatic group, substituted or unsubstituted alicyclyl, substituted or unsubstituted aryl, substituted or unsubstituted aralkyl, substituted or unsubstituted heterocyclyl and substituted or unsubstituted heteroarylalkyl;
R₂ is selected from the group formed by -NR₃R₄, -OR₃ and -SR₃, wherein R₃ and R₄ are independently selected from the group formed by H, a polymer derived from polyethylene glycol, substituted or unsubstituted non-cyclic aliphatic group, substituted or unsubstituted alicyclyl, substituted or unsubstituted heterocyclyl, substituted or unsubstituted heteroarylalkyl, substituted or unsubstituted aryl, and substituted or unsubstituted aralkyl; and R₁ or R₂ are not α-amino acids, (advantageously with the proviso that the hexapetide compound is not Ac-Asn-Glu-Met-Arg-Met-Gln-OH (SEQ ID No. 22), Ac-Gln-Glu-Met-Arg-Leu-Gln-OH (SEQ ID No. 23), or Palm-Gln-Glu-Met-Arg-Met-Asn-OH (SEQ ID No. 24), with Ac and Palm being repectively Acetyl and Palmitoyl);
whereby the dry weight content of the hexapeptide compound of formula (I) in the dedifferentiated Vitis vinifera cells enriched in hexapeptide compound of formula (I) expressed in a form free from free water is comprised between 0.1% and 8%, preferably between 0.5% and 5%.

The structures of the amino acid residues (AA1, AA2, AA3, AA4, AA5, AA6, i.e. -Asn-, -Asp-, -Gln-, -Glu-, -Arg-, -Met-, -Ile-, -Leu-, -Lys-, -Orn- and -Val- ) and their nomenclature in one and three-letter code is given, for example, in Table 1, pages 11, 12 and 13 of WO20017/100421, the content of which is incorporated by reference.

The composition can also comprise a mixture of hexapeptide compounds of formula (I). In the present specification a cosmetic composition comprising "a" or "one" hexapeptide compound of formula (I) means a cosmetic composition comprising at least one, i.e. one or more hexapeptide compounds of formula (I).

It has been observed that by using dedifferentiated elicited Vitis vinifera cells enriched with hexapeptide compound of formula (I) dispersed in a cosmetically acceptable support, a synergetic effect can be achieved for the cosmetic composition of the invention for reducing the effect of UVB irradiation, sun burning, by modulating one or more inflammation mediators.

Hexapeptide compounds of formula (I) can be prepared by the methods disclosed in WO2017/100421, more specifically by a method comprising the stages of:
- coupling of an amino acid, with the *N*-terminal end protected and the *C*-terminal end free, with an amino acid with the *N*-terminal end free and the *C*-terminal end protected or bound to a solid support;
- elimination of the protective group of the *N-*terminal end;
- repetition of the coupling sequence and elimination of the protective group of the *N* terminal end until the desired peptide sequence is obtained;
- elimination of the protective group of the *C*-terminal end or cleavage of the solid support.

Preferably, the *C*-terminal end is bound to a solid support and the process is carried out in solid phase and, therefore, comprises the coupling of an amino acid with the *N-*terminal end protected and the *C*-terminal end free with an amino acid with the *N*-terminal end free and the *C*-terminal end bound to a polymeric support; elimination of the protective group of the *N*-terminal end; and repetition of this sequence as many times as is necessary to thus obtain the compound of the desired length, finally followed by the cleavage of the synthesized compound from the original polymeric support.

The functional groups of the side chains of the amino acids are maintained conveniently protected with temporary or permanent protective groups throughout synthesis, and can be unprotected simultaneously or orthogonally to the process of cleavage of the peptide from the polymeric support.

Alternatively, solid phase synthesis can be carried out using a convergent strategy coupling a peptide with the polymeric support or with a peptide or an amino acid previously bound to the polymeric support.

The process can comprise the additional stages of deprotection of the *N*-terminal and *C*-terminal ends and/or cleavage of the peptide from the polymeric support in an indiscriminate order, using standard procedures and conditions known in the prior art, after which the functional groups of these ends can be modified. The optional modification of the *N*-terminal and *C*-terminal ends can be carried out with the peptide of formula (I) anchored to the polymeric support or once the peptide has been separated from the polymeric support.

For more details about processes suitable for the preparation of compounds of formula (I), reference can be made to the complete disclosure of WO2017/100421.

The composition of the invention has one or more of the following details or particularities :
* the dedifferentiated and elicited Vitis vinifera cells are dedifferentiated Vitis vinifera cells which have been submitted to a lighting elicitation in an air atmosphere enriched with CO₂ and having a relative humidity of more than 50%, and at a temperature from 20 to 50°C, said CO₂ enriched air atmosphere having a CO₂ volume content of 4 to 6%;
* the lighting elicitation comprises dark periods between two lighting elicitation periods.
* the lighting elicitation comprises a cycle of 80 to 120 dark periods (with a light level intensity or illuminance of less than 10 lux) at a temperature of 20°C to 50°C, advantageously about 30°C, for 30 minutes to 2 hours, advantageously 1 hour. Between two successive dark periods, the cells were submitted to a lighting elicitation intensity of 75,000 to 150,000 lux, advantageously about 100,000 lux (with lighting rays within the range 400 - 520 nm) at a temperature of 20 to 50°C, advantageously of 35°C to 45°C for 30 minutes to 2 hours.
* the hexapeptide compound of formula (I) is selected from the group consisting of:
   (i) compound(i) with AA1 : Gln, AA2 : Glu, AA3 : Met, AA4 : Arg, AA5 : Met, and AA6 : Gln, and
   (ii) compound(ii) with AA1 : Glu, AA2 : Glu, AA3 : Met, AA4 : Gln, AA5 : Arg and AA6 is Arg, and
   (iii) compound(iii) with AA1 : Gln, AA2 : Glu, AA3 : Met, AA4 : Arg, AA5 : Met and AA6 : Gln, and
   (iv) compound (ii) and compound(iii) with one to three, advantageously three of the amino acid residues AA1, AA2, AA3, AA4, AA5 and AA6 being replaced providing that:
      when AA1 is replaced, it is replaced by an amino acid selected from the group formed by Glu, Asp and Asn;
      when AA2 is replaced, it is replaced by an amino acid selected from the group formed by Gln and Asp;
      when AA3 is replaced, it is replaced by an amino acid selected from the group formed by Ile and Leu;
      when AA4 is replaced, it is replaced by an amino acid selected from the group formed by Lys and Orn;
      when AA5 is replaced, it is replaced by an amino acid selected from the group formed by Leu, Ile and Val; and
      when AA6 is replaced, it is replaced by an amino acid selected from the group formed by Asn and Glu,
   and (v) mixtures of two or more than two of said hexapeptide compounds.
* in the formula (I) of the hexapeptide compound, m,n, p and q are equal to 0,
   R₁ is selected from H, acetyl (Ac), myristoyl (Myr) and palmitoyl (Palm), R₁ being preferably acetyl, and
   R₂ is selected from -OH, -NH₂ and -NHR₃, wherein R₃ is a C₆ to C₁₈ alkyl group, preferably a hexyl (NHC₆H₁₃) or hexadecyl group (NHC₁₆H₃₃), R₂ being preferably -NH₂.
* the hexapeptide compound of formula (I) is preferably a compound with R₁ : acetyl (Ac) and R₂: -NH₂.
* the Vitis vinifera cells are cells comprising a cell wall defining an inner chamber comprising at least cytoplasm, whereby at least the cytoplasm of cells is enriched with the hexapeptide of formula (I).
* the elicited dedifferentiated Vitis vinifera cells are from Vitis vinifera flower cell origin.
* the cosmetic composition comprises a cosmetic effective amount of dedifferentiated Vitis vinifera enriched in acetyl hexapeptide for inhibiting interleukin inflammation mediators IL-1α and IL-6 at the human skin level, while modulating the human Beta Defensin 2 at the human skin level, especially moreover, for reducing and/or eliminating skin wrinkles or other skin aging effects..
* the cosmetic composition comprises glycerol forming a coating layer around the enriched elicited dedifferentiated Vitis vinfera cells. The glycerol coating comprises advantageously some hexapeptide compound(s) of formula (I). Said glycerol coating layer forms a protection layer for the enriched Vitis vinifera cells, especially when mixing the enriched Vitis vinifera cells with cosmetically acceptable solvant(s) and/or excipients.
* a combination of two or more of said details and particularities of advantageous embodiments.

The cosmetic composition object of the present invention can be prepared by means of conventional methods known by persons skilled in the art.

In case the composition comprises one or more ingredients with low water solubility, said ingredients can be solubilized in conventional cosmetically acceptable solvents such as for example ethanol, propanol or isopropanol, propylene glycol, glycerin, butylene glycol or polyethylene glycol. The enriched elicited dedifferentiated Vitis vinifera cells can also be previously incorporated in cosmetic carriers such as liposomes, milliparticles, microparticles and nanoparticles as well as in sponges, millispheres, microspheres and nanospheres, millicapsules, microcapsules and nanocapsules and liposheres. The enriched elicited dedifferentiated Vitis vinifera cells or agglomerates thereof are advantageously coated with a glycerol layer, said glycerol layer being preferably also enriched with at least one hexapeptide compound of formula (I).

The preparations or compositions of the present invention can be used in various types of formulations such as for example, and in a non-limiting sense, creams, lotions, gels, oils, liniments, serums, mousses, ointments, bars, pencils or sprays, including "leave on" and "rinse-off" formulations, and can also be incorporated by means of techniques known by persons skilled in the art to different types of solid accessories such as towelettes, hydrogels, adhesive (or non-adhesive) patches or face masks, or can be incorporated to different make-up line products such as make-up foundations, lotions, make-up removal lotions, concealers, eye shadows and lipsticks among others.

The cosmetic composition object of the present invention can be applied by means of subcutaneous injection, intradermal injection or by means of iontophoresis directly in the area of the face marked by wrinkles to achieve a greater penetration of the active ingredient. The preferred area for the application is the forehead area having expression wrinkles as well as the space between the eyebrows and the fine lines around the mouth and/or around the eyes.

The cosmetic composition of the present invention can contain additional ingredients commonly used in compositions for the care and treatment of skin, such as for example and in a non-limiting sense, emulsion agents, emollients, organic solvents, skin conditioners such as for example, humectants, alpha hydroxy acids, moisturizers, vitamins, pigments or dyes, gelling polymers, thickeners, softeners, anti-wrinkle agents, agents that can reduce or treat under-eye bags, whitening or depigmentation agents, exfoliating agents, anti-aging agents, agents capturing free radicals and/or atmospheric pollution, NO-synthase inhibiting agents, anti-oxidizing agents, anti-glycation agents, agents stimulating the synthesis of dermal or epidermal macromolecules and/or able to inhibit their degradation, such as for example agents stimulating collagen synthesis, agents stimulating elastin synthesis, agents stimulating laminin synthesis, agents inhibiting collagen degradation, agents inhibiting elastin degradation, agents stimulating fibroblast proliferation, agents stimulating keratinocyte proliferation, agents stimulating keratinocyte differentiation, agents stimulating the synthesis of lipids and components of the stratum corneum (ceramides, fatty acids etc.), skin relaxing agents, agents stimulating glycosaminoglycan synthesis, firming agents, anti-stretch mark agents, calming agents, anti-inflammatory agents, agents acting on capillary circulation and/or microcirculation, agents acting on cell metabolism, agents stimulating and/or inhibiting the synthesis of melanin, agents intended to improve the dermal-epidermal junction, preservatives, perfumes, chelating agents, plant extracts, essential oils, marine extracts, agents coming from biofermentation, mineral salts, cell extracts and sunscreens (organic or mineral photoprotection agents that are active against ultraviolet A and B rays), among others, provided that they are physically and chemically compatible with the rest of the components of the composition and especially with the peptides of general formula (I) contained in the composition of the present invention. The nature of said additional ingredients can be synthetic or natural, such as for example plant extracts.

The composition of the invention can further comprise a cosmetically effective amount of at least one further extract with anti-wrinkle and/or anti-aging activity such as for example and in a non-limiting sense, Rosa canina, Curcuma longa, Iris pallida, Theobroma cacao, Ginkgo biloba, or Dunaliella salina extracts, among others or of also at least one further synthetic compound with anti-wrinkle and/or anti-aging activity as for example and in a non-limiting sense Matrixyl^{®} marketed by Sederma, Vialo^{®} marketed by Pentapharm, Myoxinol^{™} marketed by Cognis, Algisum C^{®} or Hydroxyprolisilane CN^{®}, marketed by Exsymol, Kollaren^{®} marketed by Institut Europeen of Biologie Cellulaire, Ca²⁺ channel antagonists such as alverine, manganese or magnesium salts, certain secondary or tertiary amines, retinol and its derivatives, Coenzyme Q10 and its derivatives, boswellic acid and its derivatives or chloride channel agonists among others, mixtures thereof.

The invention further relates to a cosmetic method for inhibiting interleukin inflammation mediators IL-1α and IL-6 at the human skin level, while modulating the human Beta Defensin 2 at the skin level, the said method comprising the step of applying (possibly subcutaneously) on a portion of the skin a cosmetic composition according to the invention as disclosed here above, and the step of rubbing and/or massaging the applied cosmetic composition on the said portion of the human skin.

The cosmetic method of the invention is advantageously adapted for reducing and/or eliminating skin wrinkles (such as facial wrinkles) or other skin aging effects (such as facial aging effects).

The invention further relates to a process for the manufacture of a cosmetic composition according to the invention as disclosed here above, said process comprising at least the following steps:
- adding and mixing a hexapeptide compound of formula (I), advantageously as dry powder form, to washed and filtered humid dedifferentiated elicited Vitis vinifera cells from an in-vitro growth medium, the washed and filtered humid dedifferentiated elicited Vitis vinifera cells having a free water content of less than 30% by weight, advantageously less than 20% by weight, preferably from 1% to 15% by weight, with respect to the weight of the washed and filtered dedifferentiated elicited Vitis vinifera cells substantially free from free water; the adding and mixing steps being operated at a temperature from 5°C to 40°C, while the amount of added hexapeptide compound of formula (I) is from 0.15 to 6% of the weight of the humid dedifferentiated elicited Vitis vinifera cells free of free water;
- further malaxing without grinding the humid dedifferentiated elicited Vitis vinifera cells for at least 30minutes, advantageously from 45minutes up to 2 hours, at a temperature of 10°C to 40°C, whereby preparing humid elicited dedifferentiated Vitis vinifera cells enriched in hexapeptide compound(s) of formula (I);
- mixing the humid elicited dedifferentiated Vitis vinifera cells enriched in hexapeptide compound(s) of formula (I) with one or more cosmetic acceptable excipients and/or one or more cosmetic acceptable additives, advantageously at least with glycerol.

In said method, the enriched Vitis vinifera cells or agglomerates thereof are advantageously first mixed with glycerol, so as to form a protective glycerol coating around the cells or agglomerates thereof, before adding and mixing one or more further cosmetically acceptable compounds or excipients.

Preferred compositions of the invention will now be disclosed, as examples only, said compositions will be tested for showing its efficiency on synthetic skin. Keratinocyte cells of human origin were sown on 0.5cm² polycarbonate filters in defined and supplemented medium (modified MCDB 153). The powdered MCDB 153 media used for the preparation of the culture medium is marketed by Sigma-Aldrich, USA (https://www.sigmaaldrich.com/content/dam/sigma-aldrich/docs/Sigma/Product_Infoimation_Sheet/I/m7403pis.pdf). The cells were cultivated during 14 days at the air/liquid interface, the culture medium being changed every two days. The epidermises thus formed were used for the study completion since the 14th day of the culture.

For the testing, the active agent was present in glycerol used as cosmetically acceptable excipient for the tested cosmetic composition of the invention. The tested active agent was humid elicited dedifferentiated Vitis vinifera cells enriched with different hexapeptide compounds of formula (I), dispersed in glycerol. With respect the total weight (free from free water) of the humid elicited dedifferentiated (flower) Vitis vinifera cells enriched in hexapeptide compound of formula (I), the active agent contained :
- about 97.5% by weight of humid elicited dedifferentiated (flower) Vitis vinifera cells (CO₂ and light elicitation - see description here below)
- about 2.5 % by weight of hexapeptide compound of formula (I) in dry form

The elicited dedifferentiated (flower) Vitis vinifera cells were prepared as follow :
In a first step, Cabernet Sauvignon fower cells were cultured in-vitro, in a clean room, with a continuous lighting with an intensity of 100,000 lux, at a temperature of 30°C, in an air atmosphere with a relative humidity of 50%. The culture medium was a Murashige and Skoog medium. Other culture medium are suitable.

This first step is carried out by successively transplanting part of the in-vitro growing plant, in particular part of the root. The continuous lighting was of the type emitting more than 95% of the rays (in particular more than 99%) in the range 400 to 520 nm.

Examples of preferred hexapeptide of formula (I) which were tested are :

### SEQ ID No.1 : Acetyl hexapeptide-8

Acetyl hexapeptide-8 (CAS No 616204-22-9) is marketed under the trademark Argireline^{®}, (Ac-Glu-Glu-Met-Gln-Arg-Arg-NH₂ -SEQ ID No. 1), marketed by Lipotec (Barcelona, Spain). Argireline is used in cosmetic compositions as anti wrinkles additives.

It has the appearance of a white powder, with a purity of more than 98%. Its molecular formula is C₃₄H₆₀N₁₄O₁₂S, with a molecular weight of 888.99 grams.

The sequence listing of Acetyl hexapeptide-8 is disclosed in EP3087993 ( <170> PatentIn version 3.5; sequence listing No 1, <210> 1; <211> 6; <212> PRT; <213> Homo sapiens; <400> 1 ;
or in EP1842297 ( Sequence Id No 5)
or in EP1180524 (Sequence Id No 2). The content of said patent documents is incorporated in the present specification for dislosing the sequence listing of Acetyl Hexapeptide-8.

Artificial proteine sequences 2 to 20 with acetyl (Ac) and amino (NH2) end groups have been prepared as disclosed in WO2017/100421.

### SEQ ID No. 2 : Ac-Gln-Glu-Met-Arg-Met-Gln-NH2

### SEQ ID No. 3 : Ac-Asp-Glu-Met-Arg-Met-Gln-NH2

### SEQ ID No. 4 : Ac-Glu-Glu-Met-Arg-Met-Gln-NH2

### SEQ ID No. 5 : Ac-Gln-Gln-Met-Are-Met-Gln-NH2

### SEQ ID No. 6 : Ac-Gln-Glu-Met-Lys-Met-Gln-NH2

### SEQ ID No. 7 : Ac-Gln-Glu-Met-Arg-Met-Glu-NH2

### SEQ ID No. 8 : Ac-Gln-Glu-Met-Arg-Met-Asn-NH2

### SEQ ID No. 9 : Ac-Glu-Glu-Met-Arg-Met-Gln-NH2

### SEQ ID No. 10 : Ac-Gln-Asp-Met-Arg-Met-Glu-NH2

### SEQ ID No. 11 : Ac-Gln-Glu-Leu-Arg-Met-Gln-NH2

### SEQ ID No. 12 : Ac-Gln-Glu-Ile-Arg-Met-Gln-NH2

### SEQ ID No. 13 : Ac-Gln-Glu-Met-Arg-Ile-Gln-NH2

### SEQ ID No. 14 : Ac-Gln-Glu-Met-Arg-Leu-Gln-NH2

### SEQ ID No. 15 : Ac-Glu-Glu-Ile-Arg-Met-Gln-NH2

### SEQ ID No. 16 : Ac-Asp-Gln-Met-Arg-Met-Gln-NH2

### SEQ ID No. 17 : Ac-Glu-Gln-Met-Arg-Met-Gln-NH2

### SEQ ID No. 18 : Ac-Gln-Glu-Met-Lys-Leu-Gln-NH2

### SEQ ID No. 19 : Ac-Gln-Asp-Met-Arg-Met-Asn-NH2

### SEQ ID No. 20 : Ac-Asn-Glu-Ile-Arg-Val-Gln-NH2

### SEQ ID No. 21 : Ac-Gln-Asp-Met-Lys-Met-Asn-NH2

In a second step (elicitation step), dedifferentiated cells of step 1 were further transplanted in a Murashige and Skoog culture medium. Other in-vitro culture medium are possible. During the growing of the cells, the cells were submitted to a lighting elicitation under an air atmosphere enriched with CO₂ (CO₂ content in the CO₂ enriched air : 5% by volume) and with a relative humidity of 75%. The elicitation cycle comprises 100 dark periods (with a light intensity of less than 10 lux) at a temperature of 30°C for 1 hour. Between two successive dark periods, the cells were submitted to a lighting elicitation with an intensity of 100,000 lux (with lighting rays within the range 400 - 520 nm) at a temperature of 45°C for 1 hour.

Other possible elicitations are disclosed in EP1485064.

Said elicitation step enables to obtain elicited cells with enriched phytoalexins content, such as an increased flavanoid content (like rutin, gallic acid and isorhamnetin derivatives).

After said elicitation step, the dedifferentiated elicited Vitis vinifera cells were extracted (for example by means of a filter) and washed with water at a temperature of 20°C. After five successive washing steps, the cells were collected on a filter for reducing the free water content of the collected cells to about 20% by weight. The membrane of the cells were not damaged.

The washed humid dedifferentiated elicited Vitis vinifera cells were then mixed and malaxed with the powdered acetyl hexapeptide-8 at 20°C for 60 minutes, so as enable the hexapeptide compound (or mixture of said compounds) to migrate through the cell membrane, so as to form Vitis vinifera cells having their cytoplasm medium enriched with hexapeptide compound.

The so obtained cells are then mixed with glycerol for ensuring a protection medium or coating for the cells, before preparing the cosmetic composition. The weight content of Vitis vinifera cells witin the protective medium (such as glycerol) is for example from 0.5 to 30 % by weight, such as about 20%. Glycerol is advantageously a cosmetic excipient.

For the tests, several glycerol compositions, like Vitis vinifera cells suspensions S2,S3,S5,S6,S7 and S8 in glycerol were prepared. The tests and tested compositions or suspensions are :
* S1 (Comparative) : glycerol as such, applied at the rate of 1% by weight of the epidermis
* S2 (comparative) : glycerol with 19.5% by weight of dedifferentiated Vitis vinifera cells, applied at the rate of 1% by weight of the epidermis
* S3 (comparative) : glycerol with 19.5% by weight of elicited dedifferentiated Vitis vinifera cells, applied at the rate of 1% by weight of the epidermis
* S4 (comparative) : glycerol with 0.5% by weight of hexapeptide compound, (such as acetyl hexapeptide-8 (SEQ ID No. 1) and SEQ ID No. 2 to 21), applied at the rate of 1% by weight of the epidermis
* S5 (invention) : glycerol with 20% by weight of the dedifferentiated elicited Vitis vinifera cells enriched with acetyl hexapeptide (such as acetyl hexapeptide-8 (SEQ ID No. 1) and SEQ ID No. 2 to 21)) (about 19.5% by weight of dedifferentiated and elicited Vitis vinifera cells, and about 0.5% by weight of hexapeptide compound,) applied at the rate of 0.5% by weight of the epidermis
* S6 (invention) : glycerol with 20% by weight of the dedifferentiated elicited Vitis vinifera cells enriched with acetyl hexapeptide (such as acetyl hexapeptide-8 (SEQ ID No. 1) and SEQ ID No. 2 to 21)) (about 19.5% by weight of dedifferentiated and elicited Vitis vinifera cells, and about 0.5% by weight of hexapeptide compound,) applied at the rate of 1% by weight of the epidermis
* S7 (invention) : glycerol with 20% by weight of the dedifferentiated elicited Vitis vinifera cells enriched with acetyl hexapeptide (such as acetyl hexapeptide-8 (SEQ ID No. 1) and SEQ ID No. 2 to 21)) (about 19.5% by weight of dedifferentiated and elicited Vitis vinifera cells, and about 0.5% by weight of hexapeptide compound,) applied at the rate of 2.5% by weight of the epidermis
* S8 (invention) : glycerol with 20% by weight of the dedifferentiated elicited Vitis vinifera cells enriched with acetyl hexapeptide (such as acetyl hexapeptide-8 (SEQ ID No. 1) and SEQ ID No. 2 to 21)) (about 19.5% by weight of dedifferentiated and elicited Vitis vinifera cells, and about 0.5% by weight of hexapeptide compound,) applied at the rate of 5% by weight of the epidermis

### TEST 1 : IL-1 alpha Toxicity test without UVB radiation

Various compositions S1 to S8 were each placed on an synthetic epidermis for 24 hours. After said treatment time, the inflammation factor IL1α was measured according to the protocol described in the Kit assay IL1α of Quantikine ELISA test (R&D Systems, Minneapolis, USA) (see:
https://resources.rndsystems.com/pdfs/datasheets/dla50.pdf?v=20210423&_ga=2.2 00832941.2094027614.1619180795-613405081.1619180795 )

The results of said toxicity test for the hexapeptide compound SEQ ID No. 1 are given in the following table, the difference of IL-1 alpha level being expressed as percentage with respect to the negative control (epidermis with no added product, and not submited to UVB irradiation)

| product placed on the epidermis | difference of IL-1 alpha level with respect to the IL-1 alpha level for the negative control, expressed as percentage with respect to the negative control % |
|---|---|
| no - negative control | 0 |
| S1 | 0.5 |
| S2 | 1.0 |
| S3 | 2.2 |
| S4 | -0.9 |
| S5 (invention) | 0.5 |
| S6 (invention) | - 1.0 |
| S7 (invention) | - 1.2 |
| S8 (invention) | -0.5 |

Histological images, after HES (Hematoxylin-Eosin-Saffron) staining, of the contol epidermis and treated epidermises S1 to S8 were comparable.

The composition of the invention with acetyl hexapeptide-8 (SEQ ID No. 1) had no effect on the production of inflammation factor IL-1 alpha in physiological conditions. The composition of the invention is thus safe, and not irritating.

When reproducing said test with the hexapeptide compounds SEQ ID No.2 to Seq ID No. 21 instead of SEQ ID No. 1, it was observed that the difference of IL-1 alpha level with the composition of the invention was within the range -0.5% to +0.5% with respect to the negative control. The said compositions were thus safe and non irritating.

### TEST 2 : IL-1 alpha Activity test after UVB irradiation

The IL-1 alpha toxicity test without UVB was repeated, except that epidermises without addition of a composition (positive control) and with addition of a composition (S1 to S6) were treated for 24 hours with a UVB irradiation 100mJ/cm².

The results of said test with use of the hexapeptide compound SEQ ID No. 1 are given relative with respect to the negative control (i.e. IL-1 alpha measure for the epidermis not treated with UVB, nor with a composition).

| product placed on the epidermis UVB treated or not | IL-1 alpha measure relative to the IL-1 alpha measure for the negative control (%) |
|---|---|
| negative control | 100 |
| no UVB treatment | |
| positive control | 180 |
| No product - UVB treated | |
| S1 | 185 |
| UVB treated | |
| S2 | 178 |
| UVB treated | |
| S3 | 170 |
| UVB treated | |
| S4 | 170 |
| UVB treated | |
| S5 (invention) | 135 |
| UVB treated | |
| S6 (invention) | 120 |
| UVB treated | |
| S7 (invention) | 110 |
| UVB treated | |
| S8 (invention) | 102 |
| UVB treated | |

The composition of the invention had a clear synergic inhibition activity on the production of the IL-1 alpha inflammatory factor, following a UVB irradiation.

When reproducing said test with the hexapeptide compounds SEQ ID No.2 to Seq ID No. 21 instead of SEQ ID No. 1, it was observed that a even better synergistic inhibition activity on the production of the IL-1 alpha inflammatory factor, following a UVB irradiation was achieved with lower application rate.

### TEST 3 : IL-6 Toxicity test

Various compositions S1 to S8 were each placed on an synthetic epidermis for 24 hours. After said treatment time, the inflammation factor IL-6 was measured according to the protocol described in the Kit assay IL-6 of Quantikine ELISA test (R&D Systems, Minneapolis, USA) (see:
https://www.mdsystems.com/products/human-il-6-quantikine-elisa-kit_d6050#assay-procedure )

The results of said toxicity test with use of the hexapeptide compound SEQ ID No. 1 are given in the following table, the difference being expressed as percentage with respect to the negative control (epidermis with no added product, and not submited to UVB irradiation)

| product placed on the epidermis | Difference of IL-6 level with respect to the IL-6 level for the negative control, expressed as percentage with respect to the negative control % |
|---|---|
| no - negative control | 0.0 |
| S1 | 1.5 |
| S2 | 2.0 |
| S3 | 0.5 |
| S4 | 0.9 |
| S5 (invention) | -1.3 |
| S6 (invention) | - 5.0 |
| S7 (invention) | - 3.7 |
| S8 (invention) | - 2.1 |

The composition of the invention had no effect on the production of inflammation factor IL-6 in physiological conditions. This confirms the non irritating character of the composition of the invention.

### TEST 4 : IL-6 Activity test after UVB irradiation

The IL-6 toxicity test without UVB was repeated, except that epidermises with addition of a composition (S1 to S4 and S6 to S8) were treated for 24 hours with a UVB irradiation 100mJ/cm².

The results of said test with use of the hexapeptide compound SEQ ID No. 1 are given relative with respect to the negative control (i.e. IL-6 for the epidermis not treated with UVB, nor treated with a composition).

| product placed on the epidermis UVB treated or not | IL-6 measure relative to the IL-6 measure for the negative control (%) |
|---|---|
| negative control | 100 |
| no UVB treatment | |
| positive control | 165 |
| No product - UVB treated | |
| S1 | 160 |
| UVB treated | |
| S2 | 157 |
| UVB treated | |
| S3 | 147 |
| UVB treated | |
| S4 | 151 |
| UVB treated | |
| S6 (invention) | 136 |
| UVB treated | |
| S7 (invention) | 130 |
| UVB treated | |
| S8 (invention) | 107 |
| UVB treated | |

The composition of the invention had a clear synergistic inhibition activity on the production of the IL-6 inflammatory factor, following a UVB irradiation.

When reproducing said test with the hexapeptide compounds SEQ ID No.2 to Seq ID No. 21 instead of SEQ ID No. 1, it was observed that a even better synergistic inhibition activity on the production of the IL-6 alpha inflammatory factor, following a UVB irradiation was achieved with lower application rate.

### TEST 5 : Human beta-defensine (hBD-2) activity with and without UVB irradiation.

Various compositions S1 to S4, and S6 to S8 were each placed on an synthetic epidermis for 24 hours, with and without UVB irradiation (100mJ/cm²). After said treatment time, the peptide hBD-2 level was measured according to the protocol described in the Kit HBD-2 of Immundiagnostik AG (Bensheim, Germany) (see: http://www.immundiagnostik.com/fileadmin/pdf/beta-Defensin_2_K6500.pdf)

The results of said test with use of the hexapeptide compound SEQ ID No. 1 are given in the following table, the hBD-2 level being expressed as percentage with respect to the hBD-2 level for the negative control (epidermis with no added product, and not submited to UVB irradiation).

| product placed on epidermis | UVB treated NO / YES | hBD-2 level relative to the hBD-2 level for the negative control (%) |
|---|---|---|
| negative control no product | NO | 100 |
| S1 | NO | 102 |
| S2 | NO | 106 |
| S3 | NO | 108 |
| S4 | NO | 104 |
| S6 (invention) | NO | 115 |
| S7 (invention) | NO | 120 |
| S8 (invention) | NO | 130 |
| positive control | YES | 136 |
| S1 | YES | 135 |
| S2 | YES | 130 |
| S3 | YES | 130 |
| S4 | YES | 132 |
| S6 (invention) | YES | 112 |
| S7 (invention) | YES | 110 |
| S8 (invention) | YES | 106 |

The composition of the invention was able to control the production of hBD-2. Indeed, without UVB induction, i.e. without normal hBD-2 skin production, the production of hBD-2 was improved by 20 to 30% with respect to the negative control, while with UVB induction, i.e. generating excess hBD-2 skin production, the production of hBD-2 was inhibed. The composition of the invention had a regulating effect of the production of hBD-2,

When reproducing said test with the hexapeptide compounds SEQ ID No.2 to Seq ID No. 21 instead of SEQ ID No. 1, it was observed that a even better regulating effect on the production of hBD-2 was achieved with lower application rate.

From said tests, it appears that the composition of the invention was not skin irritating, and was effective for improving the natural production of skin hBD-2, an antimicrobial peptide exhibiting potent antimicrobial activity against Gram-negative bacteria and Candida, but not Gram-positive Staphylococcus aureus. HBD-2 is a first human defensin produced following stimulation of epithelial cells by contact with microorganisms such as Pseudomonas aeruginosa or cytokines such as TNF-alpha and IL-1 beta. Without being bound to any theory, it is expected that HBD-2 is a dynamic component of the local epithelial defense system of the skin having a role to protect surfaces from infection.

When the skin is UVB irradiated, the production of hBD-2 was regulated, for preventing its overexpression, while catalysing the inhibition of of inflammation mediators like IL-1 alpha and IL-6.

## Claims

1. Cosmetic composition comprising a cosmetic effective amount of at least dedifferentiated elicited Vitis vinifera cells homogeneously distributed in a cosmetic acceptable support, in which the dedifferentiated elicited Vitis vinifera cells, separated the one form the other and/or in cell agglomerates with a particle size of less than 0.5mm separated the one from the other, are dispersed, whereby the dedifferentiated elicited Vitis vinifera cells are enriched with at least a hexapeptide compound of the general formula (I)
R₁-Wₘ -Xₙ-AA1-AA2 -AA3-AA4-AA5-AA6-Yₚ-Z_{q}-R₂ (I)
its stereoisomers and/or its cosmetically or pharmaceutically acceptable salts, **characterized in that**:
AA1 is selected from the group formed by Gln, Glu, Asp and Asn;
AA2 is selected from the group formed by Glu, Gln and Asp;
AA3 is selected from the group formed by Met, Ile and Leu;
AA4 is selected from the group consisting of (a) Arg, Lys and Orn for any AA1, AA2 and AA3, and (b) Gln in case AA1, AA2 and AA3 are respectively Glu, Glu and Met;
AA5 is selected from the group consisting of (a) Met, Leu, Ile and Val for any AA1, AA2 and AA3, and (b) Arg in case AA1, AA2 and AA3 are respectively Glu, Glu and Met;
AA6 is selected from the group consisting of (a) Gln, Asn and Glu for any AA1, AA2 and AA3, and (b) Arg in case AA1, AA2 and AA3 are respectively Glu, Glu and Met;
W, X, Y and Z are each independently an amino acid; m, n, p and q are each independently 0 or 1 ;
R₁ is selected from the group formed by H, a polymer derived from polyethylene glycol, substituted or unsubstituted non-cyclic aliphatic group, substituted or unsubstituted alicyclyl, substituted or unsubstituted heterocyclyl, substituted or unsubstituted heteroarylalkyl, substituted or unsubstituted aryl, substituted or unsubstituted aralkyl and R₅-CO-, wherein R₅ is selected from the group formed by H, substituted or unsubstituted non-cyclic aliphatic group, substituted or unsubstituted alicyclyl, substituted or unsubstituted aryl, substituted or unsubstituted aralkyl, substituted or unsubstituted heterocyclyl and substituted or unsubstituted heteroarylalkyl;
R₂ is selected from the group formed by -NR₃R₄, -OR₃ and -SR₃, wherein R₃ and R₄ are independently selected from the group formed by H, a polymer derived from polyethylene glycol, substituted or unsubstituted non-cyclic aliphatic group, substituted or unsubstituted alicyclyl, substituted or unsubstituted heterocyclyl, substituted or unsubstituted heteroarylalkyl, substituted or unsubstituted aryl, and substituted or unsubstituted aralkyl; and R₁ or R₂ are not α-amino acids;
whereby the dry weight content of said hexapeptide compound of formula (I) in the dedifferentiated Vitis vinifera cells enriched in said hexapeptide compound of formula (I) expressed in a form free from free water is comprised between 0.1% and 8%, preferably between 0.5% and 5%.

2. The cosmetic composition of claim 1, in which the dedifferentiated and elicited Vitis vinifera cells are dedifferentiated Vitis vinifera cells which have been submitted to a lighting elicitation in an air atmosphere enriched with CO₂ and having a relative humidity of more than 50%, at a temperature from 20 to 50°C, said CO₂ enriched air atmosphere having a CO₂ volume content of 4 to 6%.

3. The cosmetic composition of claim 2, in which the lighting elicitation comprises dark periods between two lighting elicitation periods.

4. The cosmetic composition of claim 3, in which the lighting elicitation comprises a cycle of 80 to 120 dark periods with a light intensity of less than 10 lux at a temperature of 20°C to 50°C, advantageously about 30°C, for 30 minutes to 2 hours, advantageously about 1 hour, while between two successive dark periods, the cells are submitted to a lighting intensity elicitation of 75,000 to 150,000 lux, advantageously about 100,000 lux, with lighting rays within the range 400 - 520 nm, at a temperature of 20 to 50°C, advantageously of 35°C to 45°C, for 30 minutes to 2 hours.

5. The cosmetic composition of any one of the preceding claims, wherein the hexapeptide compound of formula (I) is not Ac-Asn-Glu-Met-Arg-Met-Gln-OH, Ac-Gln-Glu- Met-Arg-Leu-Gln-OH, or Palm-Gln-Glu-Met-Arg-Met-Asn-OH,

6. The cosmetic composition of any one of the preceding claims, in which the hexapeptide compound of formula (I) is selected from the group consisting of:
(i) compound(i) with AA1 : Gin, AA2 : Glu, AA3 : Met, AA4 : Arg, AA5 : Met, and AA6 : Gln, and
(ii) compound(ii) with AA1 : Glu, AA2 : Glu, AA3 : Met, AA4 : Gln, AA5 : Arg and AA6 is Arg, and
(iii) compound(iii) with AA1 : Gln, AA2 : Glu, AA3 : Met, AA4 : Arg, AA5 : Met and AA6 : Gln, and
(iv) compound (ii) and compound(iii) with one to three, advantageously three of the amino acid residues AA1, AA2, AA3, AA4, AA5 and AA6 being replaced providing that:
when AA1 is replaced, it is replaced by an amino acid selected from the group formed by Glu, Asp and Asn;
when AA2 is replaced, it is replaced by an amino acid selected from the group formed by Gln and Asp;
when AA3 is replaced, it is replaced by an amino acid selected from the group formed by Ile and Leu;
when AA4 is replaced, it is replaced by an amino acid selected from the group formed by Lys and Orn;
when AA5 is replaced, it is replaced by an amino acid selected from the group formed by Leu, Ile and Val; and
when AA6 is replaced, it is replaced by an amino acid selected from the group formed by Asn and Glu,
and (v) mixtures of two or more than two of said hexapeptide compounds.

7. The cosmetic composition of any one of the preceding claims, wherein in the formula I of the hexapeptide compound, m,n, p and q are equal to 0,
R₁ is selected from H, acetyl (Ac), myristoyl (Myr) and palmitoyl (Palm), R₁ being preferably acetyl, and
R₂ is selected from -OH, -NH₂ and -NHR₃, wherein R₃ is a C₆ to C₁₈ alkyl group, preferably a hexyl (NHC₆H₁₃) or hexadecyl group (NHC₁₆H₃₃), R₂ being preferably -NH₂.

8. The cosmetic composition of any one of the preceding claims, in which the Vitis vinifera cells are cells comprising a cell wall defining an inner chamber comprising at least cytoplasm, whereby at least the cytoplasm of cells is enriched with at least one hexapeptide compound of formula (I).

9. The cosmetic composition of any one of the preceding claims, in which the elicited dedifferentiated Vitis vinifera cells are from Vitis vinifera flower cell origin.

10. The cosmetic composition of any one of the preceding claims, which comprises at least glycerol, advantageously forming a coating layer around the elicited dedifferentiated Vitis vinifera cells, said coating layer being advantageously enriched with one or more compounds of the general formula (I).

11. The cosmetic composition of any one of the preceding claims, which comprises a cosmetic effective amount of dedifferentiated Vitis vinifera cells enriched in said hexapeptide compound(s) of formula (I) for inhibiting interleukin inflammation mediators IL-1α and IL-6 at the human skin level, while modulating the human Beta Defensin 2 at the human skin level, especially for reducing and/or eliminating skin wrinkles or other skin aging effects, said hexapeptide compound of formula (I) being preferably **characterized by** a formula I with m,n,p and q equal to 0, R₁ being acetyl and R₂ being -NH₂.

12. A cosmetic method for inhibiting interleukin inflammation mediators IL-1α and IL-6 at the human skin level, while modulating the human Beta Defensin-2 at the skin level, the said method comprising the step of applying on a portion of the skin a cosmetic composition according to any one of the claims 1 to 11, and the step of rubbing and/or massaging the applied cosmetic composition on the said portion of the human skin.

13. The cosmetic method of claim 12, for reducing and/or eliminating skin wrinkles or other skin aging effects.

14. A process for the manufacture of a cosmetic composition according to any one of the claims 1 to 11, said process comprising at least the following steps:
- adding and mixing the hexapeptide compound of formula (I), advantageously as dry powder form, to washed and filtered humid dedifferentiated elicited Vitis vinifera cells from an in-vitro growth medium, the washed and filtered humid dedifferentiated elicited Vitis vinifera cells having a free water content of less than 30% by weight, advantageously less than 20% by weight, preferably from 1% to 15% by weight, with respect to the weight of the washed and filtered dedifferentiated elicited Vitis vinifera cells substantially free from free water; the adding and mixing step being operated at a temperature from 5°C to 40°C, while the amount of added hexapeptide compound of formula (I) is from 0.15 to 6% of the weight of the humid dedifferentiated elicited Vitis vinifera cells free of free water;
- further malaxing without grinding the humid dedifferentiated elicited Vitis vinifera cells for at least 30 minutes, adavantageously from 45minutes up to 2 hours, at a temperature of 10°C to 40°C, whereby preparing humid elicited dedifferentiated Vitis vinifera cells enriched in hexapeptide compound of formula (I);
- mixing the humid elicited dedifferentiated Vitis vinifera cells enriched in hexapeptide compound of formula (I) with one or more cosmetic acceptable excipients and/or cosmetic acceptable additives, advantageously at least with glycerol.

15. The process of claim 14, in which the enriched Vitis vinifera cells or agglomerates thereof are first mixed with glycerol, so as to form a protective glycerol coating around the cells or agglomerates thereof, before adding and mixing the enriched Viis vinifera cells or agglomerates with one or more further cosmetically acceptable compounds or excipients.

## Patentansprüche

1. Kosmetische Zusammensetzung, umfassend eine kosmetische wirksame Menge an wenigstens dedifferenzierten angeregten Vitis vinifera-Zellen, die homogen in einem kosmetischen unbedenklichen Träger verteilt sind, in der die dedifferenzierten angeregten Vitis vinifera-Zellen, getrennt voneinander und/oder in Zellagglomeraten mit einer Teilchengröße kleiner 0,5 mm, die voneinander getrennt sind, dispergiert sind, wobei die dedifferenzierten angeregten Vitis vinifera-Zellen mit wenigstens einer Hexapeptidverbindung der allgemeinen Formel (I)
R₁-Wₘ-Xₙ-AA1-AA2-AA3-AA4-AA5-AA6-Yₚ-Z_{q}-R₂ (I)
, ihren Stereoisomeren und/oder ihren kosmetisch oder pharmazeutisch unbedenklichen Salzen angereichert sind, **dadurch gekennzeichnet, dass**:
AA1 aus der von Gln, Glu, Asp und Asn gebildeten Gruppe ausgewählt ist;
AA2 aus der von Glu, Gln und Asp gebildeten Gruppe ausgewählt ist;
AA3 aus der von Met, Ile und Leu gebildeten Gruppe ausgewählt ist;
AA4 aus der Gruppe bestehend aus (a) Arg, Lys und Orn für beliebige AA1, AA2 und AA3 und (b) Gln, falls AA1, AA2 bzw. AA3 Glu, Glu bzw. Met sind, ausgewählt ist;
AA5 aus der Gruppe bestehend aus (a) Met, Leu, Ile und Val für beliebige AA1, AA2 und AA3 und (b) Arg, falls AA1, AA2 bzw. AA3 Glu, Glu bzw. Met sind, ausgewählt ist;
AA6 aus der Gruppe bestehend aus (a) Gln, Asn und Glu für beliebige AA1, AA2 und AA3 und (b) Arg, falls AA1, AA2 bzw. AA3 Glu, Glu bzw. Met sind, ausgewählt ist;
W, X, Y und Z jeweils unabhängig für eine Aminosäure stehen; m, n, p und q jeweils unabhängig gleich 0 oder 1 sind;
R₁ aus der von H, einem von Polyethylenglykol abgeleiteten Polymer, einer substituierten oder unsubstituierten nicht cyclischen aliphatischen Gruppe, substituiertem oder unsubstituiertem Alicyclyl, substituiertem oder unsubstituiertem Heterocyclyl, substituiertem oder unsubstituiertem Heteroarylalkyl, substituiertem oder unsubstituiertem Aryl, substituiertem oder unsubstituiertem Aralkyl und R₅-CO- gebildeten Gruppe ausgewählt ist, worin R₅ aus der von H, einer substituierten oder unsubstituierten nicht cyclischen aliphatischen Gruppe, substituiertem oder unsubstituiertem Alicyclyl, substituiertem oder unsubstituiertem Aryl, substituiertem oder unsubstituiertem Aralkyl, substituiertem oder unsubstituiertem Heterocyclyl und substituiertem oder unsubstituiertem Heteroarylalkyl gebildeten Gruppe ausgewählt ist;
R₂ aus der von -NR₃R₄, -OR₃ und -SR₃ gebildeten Gruppe ausgewählt ist, worin R₃ und R₄ unabhängig aus der von H, einem von Polyethylenglykol abgeleiteten Polymer, einer substituierten oder unsubstituierten nicht cyclischen aliphatischen Gruppe, substituiertem oder unsubstituiertem Alicyclyl, substituiertem oder unsubstituiertem Heterocyclyl, substituiertem oder unsubstituiertem Heteroarylalkyl, substituiertem oder unsubstituiertem Aryl und substituiertem oder unsubstituiertem Aralkyl ausgewählt sind; und R₁ oder R₂ nicht für α-Aminosäuren stehen;
wobei der Trockenmassegehalt der Hexapeptidverbindung der Formel (I) in den dedifferenzierten Vitis vinifera-Zellen, die mit der Hexapeptidverbindung der Formel (I) angereichert sind, ausgedrückt in einer von freiem Wasser freien Form, zwischen 0,1 % und 8 %, bevorzugt zwischen 0,5 % und 5 % liegt.

2. Kosmetische Zusammensetzung nach Anspruch 1, bei der es sich bei den dedifferenzierten und angeregten Vitis vinifera-Zellen um dedifferenzierte Vitis vinifera-Zellen handelt, die einer Beleuchtungsanregung in einer mit CO₂ angereicherten Luftatmosphäre mit größer 50 % relative Luftfeuchtigkeit bei einer Temperatur von 20 bis 50°C unterzogen wurden, wobei die mit CO₂ angereicherte Luftatmosphäre einen CO₂-Volumengehalt von 4 bis 6 % aufweist.

3. Kosmetische Zusammensetzung nach Anspruch 2, bei der die Beleuchtungsanregung Dunkelperioden zwischen zwei Beleuchtungsanregungsperioden umfasst.

4. Kosmetische Zusammensetzung nach Anspruch 3, bei der die Beleuchtungsanregung einen Zyklus von 80 bis 120 Dunkelperioden mit einer Beleuchtungsstärke kleiner 10 lux bei einer Temperatur von 20°C bis 50°C, vorteilhaft etwa 30°C, über 30 Minuten bis 2 Stunden, vorteilhaft etwa 1 Stunde, umfasst, während zwischen zwei aufeinanderfolgenden Dunkelperioden die Zellen einer Beleuchtungsstärkeanregung von 75.000 bis 150.000 lux, vorteilhaft etwa 100.000 lux, mit Beleuchtungsstrahlen im Bereich 400 - 520 nm, bei einer Temperatur von 20°C bis 50°C, vorteilhaft 35°C bis 45°C, über 30 Minuten bis 2 Stunden unterzogen werden.

5. Kosmetische Zusammensetzung nach einem der vorhergehenden Ansprüche, wobei es sich bei der Hexapeptidverbindung der Formel (I) nicht um Ac-Asn-Glu-Met-Arg-Met-Gln-OH, Ac-Gln-Glu- Met-Arg-Leu-Gln-OH oder Palm-Gln-Glu-Met-Arg-Met-Asn-OH handelt.

6. Kosmetische Zusammensetzung nach einem der vorhergehenden Ansprüche, bei der die Hexapeptidverbindung der Formel (I) ausgewählt ist aus der Gruppe bestehend aus:
(i) Verbindung(i) mit AA1 : Gln, AA2 : Glu, AA3 : Met, AA4 : Arg, AA5 : Met und AA6 : Gin und
(ii) Verbindung(ii) mit AA1 : Glu, AA2 : Glu, AA3 : Met, AA4 : Gln, AA5 : Arg, und AA6 ist Arg, und
(iii) Verbindung(iii) mit AA1 : Gln, AA2 : Glu, AA3 : Met, AA4 : Arg, AA5 : Met und AA6 : Gin und
(iv) Verbindung (ii) und Verbindung(iii), wobei eine bis drei, vorteilhaft drei, der Aminosäurereste AA1, AA2, AA3, AA4, AA5 und AA6 ersetzt sind, unter der Voraussetzung, dass:
bei Ersetzen von AA1 dieser durch eine aus der von Glu, Asp und Asn gebildeten Gruppe ausgewählte Aminosäure ersetzt ist;
bei Ersetzen von AA2 dieser durch eine aus der von Gln und Asp gebildeten Gruppe ausgewählte Aminosäure ersetzt ist;
bei Ersetzen von AA3 dieser durch eine aus der von Ile und Leu gebildeten Gruppe ausgewählte Aminosäure ersetzt ist;
bei Ersetzen von AA4 dieser durch eine aus der von Lys und Orn gebildeten Gruppe ausgewählte Aminosäure ersetzt ist;
bei Ersetzen von AA5 dieser durch eine aus der von Leu, Ile und Val gebildeten Gruppe ausgewählte Aminosäure ersetzt ist; und
bei Ersetzen von AA6 dieser durch eine aus der von Asn und Glu gebildeten Gruppe ausgewählte Aminosäure ersetzt ist,
und (v) Gemischen von zwei oder mehr als zwei der Hexapeptidverbindungen.

7. Kosmetische Zusammensetzung nach einem der vorhergehenden Ansprüche, wobei in der Formel I der Hexapeptidverbindung m, n, p und q gleich 0 sind,
R₁ aus H, Acetyl (Ac), Myristoyl (Myr) und Palmitoyl (Palm) ausgewählt ist, wobei R₁ bevorzugt für Acetyl steht, und
R₂ aus -OH, -NH₂ und -NHR₃, wobei R₃für eine C₆- bis C₁₈-Alkylgruppe, bevorzugt eine Hexyl- (NHC₆H₁₃) oder Hexadecylgruppe (NHC₁₆H₃₃) steht, ausgewählt ist, wobei R₂ bevorzugt für -NH₂ steht.

8. Kosmetische Zusammensetzung nach einem der vorhergehenden Ansprüche, bei der es sich bei den Vitis vinifera-Zellen um Zellen handelt, die eine Zellwand umfassen, durch die eine wenigstens Cytoplasma umfassende innere Kammer definiert wird, wobei wenigstens das Cytoplasma der Zellen mit wenigstens einer Hexapeptidverbindung der Formel (I) angereichert ist.

9. Kosmetische Zusammensetzung nach einem der vorhergehenden Ansprüche, bei der die angeregten dedifferenzierten Vitis vinifera-Zellen von Vitis vinifera-Blütenzellen stammen.

10. Kosmetische Zusammensetzung nach einem der vorhergehenden Ansprüche, die wenigstens Glycerin umfasst, das vorteilhaft eine Umhüllungsschicht um die angeregten dedifferenzierten Vitis vinifera-Zellen bildet, wobei die Umhüllungsschicht vorteilhaft mit einer oder mehreren Verbindungen der allgemeinen Formel (I) angereichert ist.

11. Kosmetische Zusammensetzung nach einem der vorhergehenden Ansprüche, die eine kosmetische wirksame Menge an mit der (den) Hexapeptidverbindung(en) der Formel (I) angereicherten dedifferenzierten Vitis vinifera-Zellen umfasst, zur Hemmung der Interleukin-Entzündungsvermittler IL-1α und IL-6 auf der Ebene der menschlichen Haut bei gleichzeitiger Modulation des menschlichen Beta-Defensin 2 auf der Ebene der menschlichen Haut, vor allem zur Verringerung und/oder Beseitigung von Hautfalten oder anderen Hautalterungseffekten, wobei die Hexapeptidverbindung der Formel (I) vorzugsweise durch eine Formel I gekennzeichnet ist, in der m,n,p und q gleich 0 sind, R₁ für Acetyl und R₂ für -NH₂ steht.

12. Kosmetische Methode zur Hemmung der Interleukin-Entzündungsvermittler IL-1α und IL-6 auf der Ebene der menschlichen Haut bei gleichzeitiger Modulation des menschlichen Beta-Defensin-2 auf der Hautebene, wobei das Verfahren den Schritt, bei dem eine kosmetische Zusammensetzung gemäß einem der Ansprüche 1 bis 11 auf einen Teil der Haut aufgetragen wird, und den Schritt, bei dem die aufgetragene kosmetische Zusammensetzung auf dem Teil der menschlichen Haut eingerieben und/oder einmassiert wird umfasst.

13. Kosmetische Methode nach Anspruch 12, zur Verringerung und/oder Beseitigung von Hautfalten oder anderen Hautalterungseffekten.

14. Verfahren zur Herstellung einer kosmetischen Zusammensetzung gemäß einem der Ansprüche 1 bis 11, wobei das Verfahren wenigstens die folgenden Schritte umfasst:
- Zugeben und Mischen der Hexapeptidverbindung der Formel (I), vorteilhaft als Trockenpulverform, zu bzw. mit gewaschenen und gefilterten feuchten dedifferenzierten angeregten Vitis vinifera-Zellen aus einem In-vitro-Kulturmedium, wobei die gewaschenen und gefilterten feuchten dedifferenzierten angeregten Vitis vinifera-Zellen einen Gehalt an freiem Wasser von weniger als 30 Gew.-%, vorteilhaft weniger als 20 Gew.-%, bevorzugt 1 Gew.-% bis 15 Gew.-% in Bezug auf das Gewicht der gewaschenen und gefilterten dedifferenzierten angeregten Vitis vinifera-Zellen, die weitgehend frei von freiem Wasser sind, aufweisen, wobei der Zugabe- und Mischschritt bei einer Temperatur von 5°C bis 40°C gefahren wird, während die Menge an zugegebener Hexapeptidverbindung der Formel (I) 0,15 bis 6 % des Gewichts der feuchten dedifferenzierten angeregten Vitis vinifera-Zellen, die frei von freiem Wasser sind, beträgt;
- weitere wenigstens 30 Minuten, vorteilhaft 45 Minuten bis zu 2 Stunden, Malaxieren ohne Zermahlen der feuchten dedifferenzierten angeregten Vitis vinifera-Zellen bei einer Temperatur von 10°C bis 40°C, wodurch feuchte angeregte dedifferenzierte Vitis vinifera-Zellen hergestellt werden, die mit Hexapeptidverbindung der Formel (I) angereichert sind;
- Mischen der feuchten angeregten dedifferenzierten Vitis vinifera-Zellen, die mit Hexapeptidverbindung der Formel (I) angereichert sind, mit einem oder mehreren kosmetischen unbedenklichen Exzipienten und/oder kosmetischen unbedenklichen Zusätzen, vorteilhaft wenigstens mit Glycerin.

15. Verfahren nach Anspruch 14, bei dem die angereicherten Vitis vinifera-Zellen oder Agglomerate davon zuerst mit Glycerin gemischt werden, um so eine Glycerin-Schutzhülle um die Zellen bzw. Agglomerate davon zu bilden, bevor die angereicherten Vitis vinifera-Zellen bzw. Agglomerate mit einer/einem oder mehreren weiteren kosmetisch unbedenklichen Verbindungen oder Exzipienten zugegeben und gemischt werden.

## Revendications

1. Composition cosmétique comprenant une quantité efficace cosmétique d'au moins des cellules de Vitis vinifera dédifférenciées élicitées distribuées de manière homogène dans un support acceptable cosmétique, dans laquelle les cellules de Vitis vinifera dédifférenciées élicitées, séparées les unes des autres et/ou en agglomérats cellulaires comportant une taille de particule inférieure à 0,5 mm séparés les uns des autres, sont dispersées, moyennant quoi les cellules de Vitis vinifera dédifférenciées élicitées sont enrichies en au moins un composé hexapeptide de la formule générale (I)
R₁-Wₘ-Xₙ-AA1-AA2-AA3-AA4-AA5-AA6-Yₚ-Z_{q}-R₂ (I)
ses stéréoisomères et/ou ses sels acceptables cosmétiques ou pharmaceutiques, **caractérisée en ce que** :
AA1 est choisi dans le groupe formé par Gln, Glu, Asp et Asn ;
AA2 est choisi dans le groupe formé par Glu, Gln et Asp ;
AA3 est choisi dans le groupe formé par Met, Ile et Leu ;
AA4 est choisi dans le groupe constitué de (a) Arg, Lys et Orn pour un quelconque AA1, AA2 et AA3, et (b) Gin au cas où AA1, AA2 et AA3 sont respectivement Glu, Glu et Met ;
AA5 est choisi dans le groupe constitué de (a) Met, Leu, Ile et Val pour un quelconque AA1, AA2 et AA3, et (b) Arg au cas où AA1, AA2 et AA3 sont respectivement Glu, Glu et Met ;
AA6 est choisi dans le groupe constitué par (a) Gln, Asn et Glu pour un quelconque AA1, AA2 et AA3, et (b) Arg au cas où AA1, AA2 et AA3 sont respectivement Glu, Glu et Met ;
W, X, Y et Z sont chacun indépendamment un acide aminé ;
m, n, p et q sont chacun indépendamment 0 ou 1 ;
R₁ est choisi dans le groupe formé par H, un polymère issu d'un polyéthylène glycol, un groupe aliphatique non cyclique substitué ou non substitué, alicyclyle substitué ou non substitué, hétérocyclyle substitué ou non substitué, hétéroarylalkyle substitué ou non substitué, aryle substitué ou non substitué, aralkyle substitué ou non substitué et R₅-CO-, R₅ étant choisi dans le groupe formé par H, un groupe aliphatique non cyclique substitué ou non substitué, alicyclyle substitué ou non substitué, aryle substitué ou non substitué, aralkyle substitué ou non substitué, hétérocyclyle substitué ou non substitué et hétéroarylealkyle substitué ou non substitué ;
R₂ est choisi dans le groupe formé par -NR₃R₄, -OR₃ et - SR₃, R₃ et R₄ étant indépendamment choisis dans le groupe formé par H, un polymère issu d'un polyéthylène glycol, un groupe aliphatique non cyclique substitué ou non substitué, alicyclyle substitué ou non substitué, hétérocyclyle substitué ou non substitué, hétéroarylalkyle substitué ou non substitué, aryle substitué ou non substitué, aralkyle substitué ou non substitué ; et R₁ ou R₂ ne sont pas des acides α-aminés ;
moyennant quoi la teneur en poids sec dudit composé hexapeptide de formule (I) dans les cellules de Vitis vinifera dédifférenciées enrichies en ledit composé hexapeptide de formule (I) exprimée sous une forme exempte d'eau libre étant comprise entre 0,1 % et 8 %, préférablement entre 0,5 % et 5 %.

2. Composition cosmétique selon la revendication 1, dans laquelle les cellules de Vitis vinifera dédifférenciées et élicitées sont des cellules de Vitis vinifera dédifférenciées qui ont été soumises à une élicitation d'éclairage dans une atmosphère d'air enrichie en CO₂ et ayant une humidité relative supérieure à 50 %, à une température de 20 à 50 °C, ladite atmosphère d'air enrichie en CO₂ ayant une teneur en volume de CO₂ de 4 à 6 %.

3. Composition cosmétique selon la revendication 2, dans laquelle l'élicitation d'éclairage comprend des périodes sombres entre deux périodes d'élicitation d'éclairage.

4. Composition cosmétique selon la revendication 3, dans laquelle l'élicitation d'éclairage comprend un cycle de 80 à 120 périodes sombres comportant une intensité lumineuse inférieure à 10 lux à une température de 20 °C à 50 °C, avantageusement d'environ 30 °C, pendant 30 minutes à 2 heures, avantageusement environ 1 heure, tandis qu'entre deux périodes sombres successives, les cellules sont soumises à une élicitation d'intensité d'éclairage de 75 000 à 150 000 lux, avantageusement environ 100 000 lux, avec des rayons lumineux dans la plage de 400 - 520 nm, à une température de 20 à 50 °C, avantageusement de 35 °C à 45 °C, pendant 30 minutes à 2 heures.

5. Composition cosmétique selon l'une quelconque des revendications précédentes, le composé hexapeptide de formule (I) n'étant pas Ac-Asn-Glu-Met-Arg-Met-Gln-OH, Ac-Gln-Glu- Met-Arg-Leu-Gln-OH ou Palm-Gln-Glu-Met-Arg-Met-Asn-OH.

6. Composition cosmétique selon l'une quelconque des revendications précédentes, dans laquelle le composé hexapeptide de formule (I) est choisi dans le groupe constitué de :
(i) composé (i) avec AA1 : Gln, AA2 : Glu, AA3 : Met, AA4 : Arg, AA5 : Met et AA6 : Gln, et
(ii) composé (ii) avec AA1 : Glu, AA2 : Glu, AA3 : Met, AA4 : Gln, AA5 : Arg et AA6 est Arg, et
(iii) composé (iii) avec AA1 : Gln, AA2 : Glu, AA3 : Met, AA4 : Arg, AA5 : Met et AA6 : Gln, et
(iv) composé (ii) et composé(iii) avec un à trois, avantageusement trois des résidus d'acides aminés AA1, AA2, AA3, AA4, AA5 et AA6 qui sont remplacés à la condition que :
lorsque AA1 est remplacé, il est remplacé par un acide aminé choisi dans le groupe formé par Glu, Asp et Asn ;
lorsque AA2 est remplacé, il est remplacé par un acide aminé choisi dans le groupe formé par Gln et Asp ;
lorsque AA3 est remplacé, il est remplacé par un acide aminé choisi dans le groupe formé par Ile et Leu ;
lorsque AA4 est remplacé, il est remplacé par un acide aminé choisi dans le groupe formé par Lys et Orn ;
lorsque AA5 est remplacé, il est remplacé par un acide aminé choisi dans le groupe formé par Leu, Ile et Val ; et
lorsque AA6 est remplacé, il est remplacé par un acide aminé choisi dans le groupe formé par Asn et Glu,
et (v) des mélanges de deux ou plus de deux desdits composés hexapeptide.

7. Composition cosmétique selon l'une quelconque des revendications précédentes, dans la formule I du composé hexapeptide, m, n, p et q étant égaux à 0,
R₁ étant choisi parmi H, acétyle (Ac), myristoyle (Myr) et palmitoyle (Palm), R₁ étant préférablement acétyle, et
R₂ étant choisi parmi -OH, -NH₂ et -NHR₃, R₃ étant un groupe C₆ à C₁₈ alkyle, préférablement un groupe hexyle (NHC₆H₁₃) ou hexadécyle (NHC₁₆H₃₃), R₂ étant préférablement -NH₂.

8. Composition cosmétique selon l'une quelconque des revendications précédentes, dans laquelle les cellules de Vitis vinifera sont des cellules comprenant une paroi cellulaire définissant un compartiment interne comprenant au moins un cytoplasme, moyennant quoi au moins le cytoplasme des cellules étant enrichi avec au moins un composé hexapeptide de formule (I).

9. Composition cosmétique selon l'une quelconque des revendications précédentes, dans laquelle les cellules de Vitis vinifera dédifférenciées élicitées sont d'origine de cellules de fleurs de Vitis vinifera.

10. Composition cosmétique selon l'une quelconque des revendications précédentes, qui comprend au moins du glycérol, avantageusement formant une couche de revêtement autour des cellules de Vitis vinifera dédifférenciées élicitées, ladite couche de revêtement étant avantageusement enrichie avec un ou plusieurs composés de la formule générale (I).

11. Composition cosmétique selon l'une quelconque des revendications précédentes, qui comprend une quantité efficace cosmétique de cellules de Vitis vinifera dédifférenciées enrichies en ledit ou lesdits composés hexapeptide de formule (I) pour inhiber des médiateurs de l'inflammation de l'interleukine, IL-1α et IL-6, au niveau de la peau humaine, tout en modulant la bêta-défensine 2 humaine au niveau de la peau humaine, notamment pour la réduction et/ou l'élimination de rides de la peau ou d'autres effets du vieillissement de la peau, ledit composé hexapeptide de formule (I) étant préférablement **caractérisé par** une formule I dans laquelle m, n, p et q sont égaux à 0, R₁ étant acétyle et R₂ étant -NH₂.

12. Procédé cosmétique pour inhiber des médiateurs de l'inflammation de l'interleukine, IL-1α et IL-6, au niveau de la peau humaine, tout en modulant la bêta-défensine 2 humaine au niveau de la peau, ledit procédé comprenant l'étape d'application sur une partie de la peau d'une composition cosmétique selon l'une quelconque des revendications 1 à 11, et l'étape de frottement et/ou de massage de la composition cosmétique appliquée sur ladite partie de la peau humaine.

13. Procédé cosmétique selon la revendication 12, pour la réduction et/ou l'élimination de rides de la peau ou d'autres effets du vieillissement de la peau.

14. Processus pour la fabrication d'une composition cosmétique selon l'une quelconque des revendications 1 à 11, ledit processus comprenant au moins les étapes suivantes :
- ajout et mélange du composé hexapeptide de formule (I), avantageusement sous forme de poudre sèche, à des cellules de Vitis vinifera dédifférenciées élicitées humides lavées et filtrées provenant d'un milieu de croissance *in vitro,* les cellules de Vitis vinifera dédifférenciées élicitées humides lavées et filtrées ayant une teneur en eau libre inférieure à 30 % en poids, avantageusement inférieure à 20 % en poids, préférablement de 1 % à 15 % en poids, par rapport au poids des cellules de Vitis vinifera dédifférenciées élicitées lavées et filtrées sensiblement exemptes d'eau libre ; l'étape d'ajout et de mélange étant réalisée à une température de 5 °C à 40 °C, tandis que la quantité de composé hexapeptide de formule (I) ajoutée étant de 0,15 à 6 % du poids des cellules de Vitis vinifera dédifférenciées élicitées humides exemptes d'eau libre ;
- en outre malaxage sans broyage des cellules de Vitis vinifera dédifférenciées élicitées humides pendant au moins 30 minutes, avantageusement pendant 45 minutes jusqu'à 2 heures, à une température de 10 °C à 40 °C, préparant ainsi des cellules de Vitis vinifera dédifférenciées élicitées humides enrichies en composé hexapeptide de formule (I) ;
- mélange des cellules de Vitis vinifera dédifférenciées élicitées humides enrichies en composé hexapeptide de formule (I) avec un ou plusieurs excipients acceptables cosmétiques et/ou additifs acceptables cosmétiques, avantageusement au moins avec du glycérol.

15. Procédé selon la revendication 14, dans lequel les cellules de Vitis vinifera enrichies ou des agglomérats de celles-ci sont d'abord mélangé(e)s avec du glycérol, de sorte à former un revêtement de glycérol protecteur autour des cellules ou des agglomérats de celles-ci, avant d'ajouter et de mélanger les cellules de Vitis vinifera enrichies ou les agglomérats avec un ou plusieurs composés ou excipients supplémentaires acceptables cosmétiques.
